# EUROPEAN PATENT SPECIFICATION

(11) **EP 2 175 039 B1**
(45) Date of publication and mention of the grant of the patent: **16.11.2011**
(21) Application number: 09167120.6
(22) Date of filing: 03.08.2009
(51) Int. Cl.: C12Q 1/68

(54) **Analysis for the genetic disposition for left-sided abomasal displacement in cattle**
Analyse zur genetischen Veranlagung der linksseitigen Labmagenverlagerung bei Rindern
Analyse de la disposition génétique pour le déplacement à gauche de la caillette chez le bétail

(30) Priority: 01.08.2008 EP 08161633
(43) Date of publication of application: 14.04.2010
(73) Proprietor: Stiftung Tierärztliche Hochschule Hannover, 30559 Hannover (DE)
(72) Inventor: Distl, Ottmar Prof. Dr. Dr., 30559 Hannover (DE); Mömke, Stefanie Dr., 30173 Hannover (DE)
(74) Representative: Taruttis, Stefan Georg

(56) References cited:
- CHEBEL R C ET AL: "Leptin genotype is associated with lactation performance and health of Holstein cows" JOURNAL OF DAIRY SCIENCE, vol. 91, no. 7, July 2008 (2008-07), pages 2893-2900, XP002568426 ISSN: 0022-0302
- ONTSOUKA E C ET AL: "mRNA expression of motility-mediating receptors from the abomasum to the spiral colon of healthy cows and of cows suffering from left-sided abomasal displacement" POULTRY SCIENCE, CHAMPAIGN, IL, US, vol. 86, no. Suppl. 1, 1 January 2007 (2007-01-01), page 469, XP009129400 ISSN: 0032-5791
- RICKEN M ET AL: "Genetische Analyse der Prävalenz von Labmagenverlagerung und deren Beziehung zu Milchleistungsmerkmalen bei Deutschen Holstein Kühen // Genetic analysis of the prevalence of abomasal displacement and its relationship to milk output characteristics in German Holstein cows" DTW, DEUTSCHE TIERAERZTLICHE WOCHENSCHRIFT,, vol. 111, no. 9, 1 September 2004 (2004-09-01), pages 366-370, XP009129419 ISSN: 0341-6593
- KOLBEHDARI D ET AL: "A whole-genome scan to map quantitative trait loci for conformation and functional traits in Canadian Holstein Bulls" JOURNAL OF DAIRY SCIENCE, vol. 91, no. 7, July 2008 (2008-07), pages 2844-2856, XP002568427 ISSN: 0022-0302

### SEQUENCE LISTING

<110> Stiftung Tierärztliche Hochschule Hannover
   <120> Analysis for the genetic disposition for left sided abomasal displacement in cattle
<130> T1008-N-EP
<160> 28
<170> PatentIn version 3.5
<210> 1
   <211> 21
   <212> DNA
   <213> Artificial Sequence
<220>
   <223> /note = "synthetic sequence, marker SEQ-ID NO 1"
<400> 1
   ggtatataag ggcccgtcgg a 21
<210> 2
   <211> 20
   <212> DNA
   <213> Artificial Sequence
<220>
   <223> /note = "synthetic sequence, marker SEQ-ID NO 2"
<400> 2
   agctctctcc acttgtcttc 20
<210> 3
   <211> 19
   <212> DNA
   <213> Artificial Sequence
<220>
   <223> /note = "synthetic sequence, marker SEQ-ID NO 3"
<400> 3
   cgtgcggccg tccacatcc 19
<210> 4
   <211> 20
   <212> DNA
   <213> Artificial Sequence
<220>
   <223> /note = "synthetic sequence, marker SEQ-ID NO 4"
<400> 4
   tgcccctccg ggccggcact 20
<210> 5
   <211> 20
   <212> DNA
   <213> Artificial Sequence
<220>
   <223> /note = "synthetic sequence, marker SEQ-ID NO 5"
<400> 5
   cccaacaggc caaagaaccc 20
<210> 6
   <211> 20
   <212> DNA
   <213> Artificial Sequence
<220>
   <223> /note = "synthetic sequence, marker SEQ-ID NO 6"
<400> 6
   cccacccccg taccaccata 20
<210> 7
   <211> 21
   <212> DNA
   <213> Artificial Sequence
<220>
   <223> /note = "synthetic sequence, marker SEQ-ID NO 7"
<400> 7
   actgggctgc gctgcaggag 21
<210> 8
   <211> 21
   <212> DNA
   <213> Artificial Sequence
<220>
   <223> /note = "synthetic sequence, mutant marker SEQ-ID NO 8"
<400> 8
   ggtatataag gacccgtcgg a 21
<210> 9
   <211> 20
   <212> DNA
   <213> Artificial Sequence
<220>
   <223> /note = "synthetic sequence, mutant marker SEQ-ID NO 9"
<400> 9
   agctctctcc gcttgtcttc 20
<210> 10
   <211> 20
   <212> DNA
   <213> Artificial Sequence
<220>
   <223> /note = "synthetic sequence, mutant marker SEQ-ID NO 10"
<400> 10
   cgtgcggccc gtccacatcc 20
<210> 11
   <211> 20
   <212> DNA
   <213> Artificial Sequence
<220>
   <223> /note = "synthetic sequence, mutant marker SEQ-ID NO 11"
<400> 11
   tgcccctccc ggccggcact 20
<210> 12
   <211> 21
   <212> DNA
   <213> Artificial Sequence
<220>
   <223> /note = "synthetic sequence, mutant marker SEQ-ID NO 12"
<400> 12
   cccaacaggc caaaagaacc c 21
<210> 13
   <211> 20
   <212> DNA
   <213> Artificial Sequence
<220>
   <223> /note = "synthetic sequence, mutant marker SEQ-ID NO 13"
<400> 13
   cccaccccca taccaccata 20
<210> 14
   <211> 21
   <212> DNA
   <213> Artificial Sequence
<220>
   <223> /note = "synthetic sequence, mutant marker SEQ-ID NO 14"
<400> 14
   actgggctgt gctgcaggag 21
<210> 15
   <211> 17
   <212> DNA
   <213> Artificial Sequence
<220>
   <223> /note = "synthetic sequence, primer SEQ-ID NO 15"
<400> 15
   gccaggccct gggttgg 17
<210> 16
   <211> 20
   <212> DNA
   <213> Artificial Sequence
<220>
   <223> /note = "synthetic sequence, primer SEQ-ID NO 16"
<400> 16
   tttctacgag ccccaggaat 20
<210> 17
   <211> 17
   <212> DNA
   <213> Artificial Sequence
<220>
   <223> /note = "synthetic sequence, primer SEQ-ID NO 17"
<400> 17
   gccaggccct gggttgg 17
<210> 18
   <211> 20
   <212> DNA
   <213> Artificial Sequence
<220>
   <223> /note = "synthetic sequence, primer SEQ-ID NO 18"
<400> 18
   tttctacgag ccccaggaat 20
<210> 19
   <211> 20
   <212> DNA
   <213> Artificial Sequence
<220>
   <223> /note = "synthetic sequence, primer SEQ-ID NO 19"
<400> 19
   tcccagctcc agcaagtcta 20
<210> 20
   <211> 20
   <212> DNA
   <213> Artificial Sequence
<220>
   <223> /note = "synthetic sequence, primer SEQ-ID NO 20"
<400> 20
   gacacaccca cttatgggat 20
<210> 21
   <211> 19
   <212> DNA
   <213> Artificial Sequence
<220>
   <223> /note = "synthetic sequence, primer SEQ-ID NO 21"
<400> 21
   ttgttggccc ttccctttg 19
<210> 22
   <211> 20
   <212> DNA
   <213> Artificial Sequence
<220>
   <223> /note = "synthetic sequence, primer SEQ-ID NO 22"
<400> 22
   gacacaccca cttatgggat 20
<210> 23
   <211> 21
   <212> DNA
   <213> Artificial Sequence
<220>
   <223> /note = "synthetic sequence, primer SEQ-ID NO 23"
<400> 23
   aatcgtgtaa aactcacctt c 21
<210> 24
   <211> 20.
   <212> DNA
   <213> Artificial Sequence
<220>
   <223> /note = "synthetic sequence, primer SEQ-ID NO 24"
<400> 24
   tgtgaagttg tctgagatgc 20
<210> 25
   <211> 20
   <212> DNA
   <213> Artificial Sequence
<220>
   <223> /note = "synthetic sequence, primer SEQ-ID NO 25"
<400> 25
   aacccccggt gattagactc 20
<210> 26
   <211> 20
   <212> DNA
   <213> Artificial Sequence
<220>
   <223> /note = "synthetic sequence, primer SEQ-ID NO 26"
<400> 26
   agggattaac ggcttgctct 20
<210> 27
   <211> 20
   <212> DNA
   <213> Artificial Sequence
<220>
   <223> /note = "synthetic sequence, primer SEQ-ID NO 27"
<400> 27
   aacccccggt gattagactc 20
<210> 28
   <211> 20
   <212> DNA
   <213> Artificial Sequence
<220>
   <223> /note = "synthetic sequence, primer SEQ-ID NO 28"
<400> 28
   agggattaac ggcttgctct 20

## Description

The present invention relates to a process for analysis of the genetic disposition in individual cattle, especially in German Holstein cows, to develop left-sided abomasal displacement (LDA), and to identify individuals carrying the hereditary trait, i.e. an allele coupled to LDA. In accordance with the process, which is based on the genetic analysis of cattle, the invention also relates to the use of the relevant genetic markers in the analytical process, to the use of oligonucleotides which are specific for a DNA or RNA section containing the relevant marker, e.g. the allele coupled to LDA, e.g. for use of oligonucleotides as primers in PCR amplification using genomic DNA of the individual bovine, as well as to the alleles of markers indicative of presence or absence of genetic predisposition for LDA, and to the oligonucleotides usable as primers for amplifying the markers coupled to LDA.

The preferred race of cattle for the analytical process is German Holstein and related races, e.g. Holsteins in all other European countries, including French, British, Italian, Dutch and Danish Holsteins, cross-breds with Holsteins, and cattle families with at least one ancestor or founder animal of the aforementioned races, especially of German Holstein. The analytical process of the invention is based on the detection of at least one genetic aberration in at least one genetic marker found to be indicative of the genetic disposition for LDA. Further, the invention provides a process for determination of the individual risk of a bovine to develop phenotypic LDA, as well as a process for determination of the propensity of an individual bovine to pass on the genetic trait for phenotypic LDA to offspring, e.g. for selecting breeding animals.

### State of the art

Today, LDA, which only affects cows, but not bulls, is only phenotypically detected in cows. Accordingly, there is no direct method for analysis available to determine whether a bull, e.g. a bull used for artificial insemination (AI) is a carrier of an allele for LDA.

Kolbehdari et al. in journal of dairy science, 2844 - 2856 (2008) describe an SNP which is located in exon 2 of the leptin gene, which SNP is said to correlate with an increased incidence of LDA.

### Objects of the invention

In view of the shortcomings of prior art, it is an object of the present invention to provide a genetic test for analysis of cattle, including cows and bulls, to determine their genetic predisposition to pass on an allele causing LDA, and to develop LDA themselves.

### General description of the invention

The invention achieves the above-mentioned objects by the subject-matter as defined in the claims, and especially by providing a method for genetic analysis of a bovine, especially of German Holstein cattle, which is suitable to predict the individual risk of the bovine to develop phenotypic LDA and for the determination of the individual risk of an animal for hereditary transmission of an allele causing LDA, preferably by identification of mutations in both alleles of markers of the invention, e.g. homozygous mutant alleles. The genetic analysis identifies the presence of alleles of genetic markers that have been found to be coupled to LDA, i.e. identification of the presence of alleles of genetic markers that have been found to be present in cows affected by phenotypic LDA, whereas another allele of the marker was found to be associated with a non-LDA phenotype. The markers and processes of the invention are especially suitable for the determination of the genetic propensity of male cattle to pass on the trait for LDA, because to-date, no reliable method is available for predicting the genotype of male cattle in respect of LDA.

As used herein, the wild-type alleles of the markers are not associated with LDA and, accordingly, the presence of both marker alleles in the wild-type sequence (TIHO_LDA 1 to TIHO_LDA 7) indicates an LDA-free genome and animal, respectively, whereas mutant sequences of the least one of the markers increase the risk for LDA, both for one copy of a mutant allele (mutant marker is heterozygous), and for two copies of a mutant allele of the marker (mutant marker is homozygous). Generally, nucleotide sequences are given from 5' to 3'.

The analytical method identifies alleles of genetic markers which could be shown to be coupled to genetic factors contributing to the LDA phenotype. Accordingly, the invention also provides the genetic markers, aberrations of which from the wild-type sequence are indicative of genetic factors coupled to the LDA-phenotype. Further, specific oligonucleotide sequences are provided that can be used as primers in PCR reactions for specific amplification of DNA fragments containing the genetic markers. The analytical method is based on the determination of an aberration, i.e. a mutation, especially a single nucleotide polymorphism (SNP), in at least one of the genetic markers. Markers that are relevant for the genetic disposition to develop LDA are given in Table 1 below, wherein the wild-type sequence of the marker indicates a genetic factor not contributing to LDA, whereas aberrations in the genetic markers indicate a contributing factor to the genetic disposition for LDA. Exemplary SNPs in the markers are given, which could be shown to be coupled to a LDA at least in German Holstein cows, including black and white, and red and white. As the SNPs are determinable independent from their localisation on the coding or on the non-coding strand of genomic DNA, the invention also relates to the nucleic acid sequences which are reverse complementary to the nucleic acid sequences given herein, and to the use of nucleic acid sequences which are reverse complementary to the nucleic acid sequences given herein in the processes of the invention.

The wild-type alleles of the marker sequences, which are termed TIHO_LDA 1 (Seq.-ID No. 1), TIHO_LDA 2 (Seq.-ID No. 2), TIHO_LDA 3 (Seq.-1D No. 3), TIHO_LDA 4 (Seq.-ID No. 4), TIHO_LDA 5 (Seq.-ID No. 5), TIHO_LDA 6 (Seq.-ID No. 6), and TIHO_LDA 7 (Seq.-ID No. 7) have been found to be coupled with a non-LDA phenotype. In contrast, mutant alleles of these wild-type markers could be identified by an SNP, which mutant alleles have been found to be indicative of the genetic disposition for LDA, for example the following mutant sequences: for TIHO_LDA 1 Seq.-ID No. 8 (mut-TIHO_LDA1), for TIHO_LDA 2 sequence Seq ID No. 9 (mut-TIHO_LDA 2), carrying an A to G base exchange, for TIHO_LDA 3, mutant sequence Seq ID No. 10 (mut-TIHO_LDA 3), carrying an insertion of the C, for TIHO_LDA 4, mutant sequence Seq ID No. 11 (mut-TIHO_LDA 3), carrying a base exchange of G to C, for TIHO-LDA 5, mutant sequence Seq ID No. 12 (mut-TIHO_LDA 5), carrying an insertion of A, for TIHO_LDA 6 mutant sequence Seq ID No. 13 (mut-TIHO_LDA 6), carrying a base exchange from G to A, and for the marker TIHO_LDA 7 mutant sequence Seq ID No. 14 (mut-TIHO_LDA 7), carrying a base exchange from C to G. An overview of the markers and their wild-type sequences and exemplary mutant sequences which are coupled to LDA is given in Table 1.

**Table 1: Marker sequences for non-LDA (wild-type) and LDA-coupled alleles**

| Marker | Marker |
|---|---|
| Wild-type sequence | Mutant sequence |
| TIHO_LDA1 | mut-TIHO_LDA1 |
| GGTATATAAGGGCCCGTCGGA | GGTATATAAGGACCCGTCGGA |
| (Seq.-ID No. 1) | (Seq.-ID No. 8) |
| TIHO_LDA2 | mut-TIHO_LDA2 |
| AGCTCTCTCCACTTGTCTTC | AGCTCTCTCCGCTTGTCTTC |
| (Seq.-ID No. 2) | (Seq.-ID No. 9) |
| TIHO_LDA3 | mut-TIHO_LDA3 |
| CGTGCGGCC_GTCCACATCC | CGTGCGGCCCGTCCACATCC |
| (Seq.-ID No. 3) | (Seq.-ID No. 10) |
| TIHO_LDA4 | mut-TIHO_LDA4 |
| TGCCCCTCCGGGCCGGCACT | TGCCCCTCCCGGCCGGCACT |
| (Seq.-ID No. 4) | (Seq.-ID No. 11) |
| TIHO_LDA5 | mut-TIHO_LDA5 |
| CCCAACAGGCC_AAAGAACCC | CCCAACAGGCCAAAAGAACCC |
| (Seq.-ID No. 5) | (Seq.-1D No. 12) |
| TIHO_LDA6 | mut-TIHO_LDA6 |
| CCCACCCCCGTACCACCATA | CCCACCCCCATACCACCATA |
| (Seq.-ID No. 6) | (Seq.-ID No. 13) |
| TIHO_LDA7 | mut-TIHO_LDA7 |
| ACTGGGCTGCGCTGCAGGAGG | ACTGGGCTGTGCTGCAGGAGG |
| (Seq.-ID No. 7) | (Seq.-ID No. 14) |

In the analytical process of the invention, aberrations from the wild-type sequences of at least one of the markers of Seq ID Nos. 1 to 7, preferably in both alleles, indicate a genome coupled to an increased genetic propensity for the individual cow to develop phenotypic LDA, as well as to the individual bovine to pass the genetic trait for LDA to its offspring.

Further, the invention provides for an estimation of the propensity of an individual to develop LDA. Since the analytical method of the invention is based on the identification of mutations in the wild-type alleles of the genetic markers of the invention, there is also provided an estimation of the propensity of an individual bovine to pass on the genetic trait for LDA to its offspring, and, accordingly, the genetic influence from a parent individual onto the risk for a descendant to develop LDA can be calculated.

It is preferred to analyze at least one, preferably two or more, more preferably at least 3, most preferably all of the genetic markers for LDA, to increase the confidence of the estimation of the risk for LDA in an individual cow, or of the hereditary contribution of an animal to its offspring to develop LDA or carry the genetic predisposition to further pass on an LDA-affected genotype. Among the markers of the invention, TIHO_LDA 3 has been found to be the one most strongly associated with LDA, and therefore the invention can in its embodiments relate to T1HQ_LDA 3 and its use in the processes of the invention, with markers TIHO_LDA 1, TIHO_LDA 2, and TIHO_LDA 4 to TIHO_LDA 7 preferred as additional markers. As it has been found that animals carrying the mutant allele of the marker TIHO_LDA 2 or the mutant allele of the marker TIHO_LDA 3, homozygously, are at a high risk to develop phenotypic LDA, the markers TIHO_LDA 2 and/or TIHO_LDA 3 are especially preferred in the invention. More preferably, invention relates to the group containing or consisting of markers TIHO_LDA1, TIHO_LDA 2 and TIHO_LDA 3, and its use in the analysis. In addition to or in the alternative to markers TIHO_LDA 2 and/or TIHO_LDA 3, the invention relates to markers TIHO_LDA I in combination with TIHO_LDA 4 to TIHO_LDA 7, preferably to markers TIHO_LDA 1 in combination with TIHO_LDA 3 to TIHO_LDA 7.

Therefore the genetic test of the invention allows a prediction of the probability of a bull to pass on the genetic trait for LDA to its offspring, which is of particular relevance for the selection of future sires, especially of bulls producing sperm for artificial insemination, and for the selection of dams of these sires, which usually have a high number of descendants. For the dams, an advantage of the genetic analysis of the invention lies in fact that their genetic disposition for LDA can be determined at an early age.

Accordingly, the disclosure also includes a process for breeding cattle, which process includes the step of analysing the genotype of an individual by a method according to the invention, e.g. including the identification of at least one of the wild-type markers.

It has been found that the markers of the invention are all located within the bovine motilin (MLN) gene, and further, the markers were found to be located within the non-translated sections of the motilin gene, especially in the promoter region of the motilin gene, in the 5'UTR of the motilin gene, and in introns of the motilin gene, more especially in intron I, intron 2 and intron 4, respectively. It is therefore concluded that the effect of the mutant marker alleles is an altered, e.g. a reduced expression of the motilin gene, which altered expression results in an LDA phenotype.

The nucleotide sequences of markers TIHO_LDA 2 to TIHO_LDA 7, and of primer sequences given for amplification of gene portions comprising these markers, can be found in the motilin gene sequence as given under accession No. (ENSBTAG00000015550, Ensembl), with marker TIHO_LDA 1 given herein as the reverse complementary nucleotide sequence in relation to NCBI No. 280860.

During the preparation of the present invention, further polymorphisms in the motilin gene have been identified. However, other polymorphisms have been found not to be coupled to LDA, and, accordingly, only alleles of TIHO_LDA 1 to TIHO_LDA 7, e.g. their mutant alleles as described herein, are coupled to LDA.

### Detailed description of the invention

The invention is now described in greater detail with reference to the figures, which show the results of gel-electrophoretical analyses of a PCR amplification product containing the respective marker, namely in
- Figure 1 for TIHO LDA 1 using restriction with ApaI,
- Figure 2 for TIHO_LDA 2 using restriction with XcmI,
- Figure 3 for TIHO LDA 3 using electrophoretic size-separation,
- Figure 4 for TIHO_LDA 4 by restriction with BsiBI,
- Figure 5 for TIHO_LDA 5 using electrophoretic size-separation,
- Figure 6 for TIHO_LDA 6 by restriction with CviQI, and
- Figure 7 for TIHO_LDA 7 by restriction with HhaI.

The markers were identified by analysis of 1166 German Holstein cows, which were chosen from more than 3,000 cows affected by LDA, and more than 2,000 control cows without LDA.

For estimating the genetic predisposition of an animal to develop LDA or to pass on the genetic disposition for LDA to offspring, at least one marker from the group consisting of makers TIHO_LDA 1, TIHO_LDA 2, TIHO_LDA 3, TIHO_LDA 4, TIHO_LDA 5, TIHO_LDA 6 and TIHO_LDA 7 is analysed for an aberration. Preferably, marker TIHO_LDA 3 is included in the analysis, because its mutant allele indicates a high risk for LDA, e.g. the genotype for marker TIHO_LDA 3 insC/insC or insC/wild-type identifies animals having a high risk to develop LDA.

For markers TIHO_LDA 1 and TIHO_LDA 2 as well as TIHO_LDA 4, TIHO_LDA 5, TIHO_LDA 6 and TIHO_LDA 7, it is preferred to analyze these markers jointly, to determine a joint haplotype for markers TIHO_LDA 1, TIHO_LDA 2, and TIHO_LDA 4 to TIHO_LDA 7. However, the risk for LDA can be determined with a high accuracy by analysing only TIHO_LDA 3 for an aberration, as mutations in TIHO_LDA 3 are coupled with a high risk of LDA.

Preferably, at least one of the markers is analysed for the presence of a mutation, including preferably at least TIHO_LDA 3. It is preferred that the analysis includes at least two markers, at least three or more markers, preferably all markers from TIHO_LDA 1, TIHO_LDA 2, TIHO_LDA 4, TIHO_LDA 5, TIHO_LDA 6, and TIHO_LDA 7, for inferring the individual risk for LDA, or for inferring the propensity to pass on the genetic trait for LDA to offspring. Most preferably, all markers are included in the analysis of a bovine.

For analysis of the markers for identification of mutant sequences, especially SNPs, sequencing of the marker can be used, or an electrophoretic size-separation, e.g. of extension products of an oligonucleotide primer (preferably labelled) generated by a DNA-polymerase, or an analysis by restricting DNA containing the marker using a restriction enzyme, the activity of which enzyme is dependent on the presence or absence of a mutation in the marker, e.g. RFLP. Analytical methods for identification of mutant sequences in the markers are given in following Table 2 for the mutant sequences of the markers indicated in Table 1.

**Table 2: Exemplary analysis of PCR products for the detection of a mutation in one of the markers TIHO LDA**

| Marker (wild-type) | Genotyping method | fragments (bp) wild-type (homozygous) | fragments (bp) wild-type / mutant (heterozygous) | fragments (bp) mutant (homozygous) | Restriction temperature and buffers |
|---|---|---|---|---|---|
| TIHO_LDA1 | enzyme: ApaI | 100,374 | 100,374, 474 | 474 | 25 °C NEB4, BSA |
| TIHO_LDA2 | enzyme: XcmI | 127,347 | 127,347, 474 | 474 | 37 °C NEB2 |
| TIHO_LDA3 | electrophoretic size-separation | 154 | 154/155 | 155 | |
| TIHO_LDA4 | enzyme: BsiEI | 29,485 | 29, 143, 342, 485 | 29, 143, 342 | 60 °C NEB2, BSA |
| TIHO_LDA5 | electrophoretic size-separation | 152 | 152/153 | 153 | |
| TIHO_LDA6 | enzyme: CviQI | 64/105/175 | 64/105/175/ 239 | 105/239 | 25°C NEB3, BSA |
| TIHO_LDA7 | enzyme: HhaI | 77/133/134 | 77/133/134/ 210 | 134 / 210 | 37°C NEB4, BSA |

Preferably, the analysis of the marker sequences is based on DNA amplificates produced by PCR on a DNA sample isolated from an individual bovine, preferably on total genomic DNA. Specific primers, which are preferably used pairwise as indicated below, are given in Table 3 for the marker-specific amplification from total genomic DNA of an individual cow or bull. For sequencing and electrophoretic size-separation, at least one labelled (IRD 800) oligonucleotide was used in a PCR-sequencing reaction using ddNTPs and a LI-COR 4300 automated sequencer (LI-COR, Lincoln, USA).

**Table 3: Primers for pairwise use for specific amplification of markers in a PCR reaction, including preferred annealing temperature (AT) and size of the amplificate (P).**

| Marker | Primer f (5' > 3') | Primer r (5' > 3') | AT (°C) | P (bp) |
|---|---|---|---|---|
| TIHO_LDA 1 | LDA 1-f | LDA 1-r | 60 | 474 |
| | GCCAGGCCCTGGGTTGG (Seq.-ID No. 15) | TTTCTACGAGCCCCAG GAAT (Seq.-ID No. 16) | | |
| TIHO_LDA 2 | LDA 2-f | LDA 2-r | 60 | 474 |
| | GCCAGGCCCTGGGTTGG (Seq.-ID No. 17) | TTTCTACGAGCCCCAG GAAT (Seq.-ID No. 18) | | |
| TIHO_LDA 3 | LDA 3-f | LDA 3-r | 60 | 154 |
| | TCCCAGCTCCAGCAAGTC TA* (Seq.-ID No. 19) | GACACACCCACTTATG GGAT (Seq.-ID No. 20) | | |
| TIHO_LDA 4 | LDA 4-f | LDA 4-r | 58 | 514 |
| | TTGTTGGCCCTTCCCTTT G (Seq.-ID No. 21) | GACACACCCACTTATG GGAT (Seq.-ID No. 22) | | |
| TIHO_LDA 5 | LDA 5-f | LDA 5-r | 60 | 152 |
| | AATCGTGTAAAACTCACC TTC (Seq.-ID No. 23) | TGTGAAGTTGTCTGAG ATGC* (Seq.-ID No. 24) | | |
| TIHO_LDA 6 | LDA 6-f | LDA 6-r | 60 | 344 |
| | AACCCCCGGTGATTAGAC TC (Seq.-ID No. 25) | AGGGATTAACGGCTT GCTCT (Seq.-ID No. 26) | | |
| TIHO_LDA 7 | LDA 7-f | LDA 7-r | 60 | 344 |
| | AACCCCCGGTGATTAGAC TC (Seq.-ID No. 27) | AGGGATTAACGGCTT GCTCT (Seq.-ID No. 28) | | |

| | | | | |
|---|---|---|---|---|
| * preferably fluorescence-labelled for detection in sequencing reaction | | | | |

In Table 3, oligonucleotide primers are named in accordance with the respective TIHO_LDA marker which is specifically amplified, as LDA-f, including the number of the TIHO_LDA marker, with f indicating a forward-primer and r indicating the reverse-primer, which are preferably used pairwise with the same numbers, e.g. LDA 1-f paired with LDA 1 -r. Preferred reaction conditions for PCR amplification using these oligonucleotide primers are given in Table 4. For sequencing, it is preferred to use fluorescence - labelled primers, e.g. for use in automatic detection of a fluorescence - labelled sequencing primer. As a sequencing primer, one of the primers of Table 3 for the respective amplification of a fragment containing the marker can be used.

**Table 4: PCR conditions for specific amplification of markers using the primers as indicated in Table 3, for subsequent analysis of the PCR amplificate by RFLP or sequencing**

| | PCR (restriction and MegaBACE) | | | | PCR (IRD-labelled primers for LI-COR sequencing) | | |
|---|---|---|---|---|---|---|---|
| Step 1 | 94 °C | 4:00* | | | 94 °C | 4:00* | |
| Step 2 | 94 °C | 0:30* | Cycle start | | 94 °C | 0:30* | Cycle start |
| Step 3 | AT (Table 3) | 1:00* | | | AT (Table 3) | 0:50* | |
| Step 4 | 72 °C | 1:20* | Cycle end, 37 x | | 72 °C | 0:50* | Cycle end, 37 x |
| Step 5 | 72 °C | 5:00* | | | 72 °C | 3:00* | |
| Step 6 | 4 °C | 10:00* | | | 4°C | 10:00* | |

| | | | | | | | |
|---|---|---|---|---|---|---|---|
| *=min | | | | | | | |

Using the genome sequences of Btau 4.0, it has been found that markers TIHO_LDA 1 to TIHO_LDA 7 are located in the bovine genome in untranslated regions, e.g. the 5'UTR, promoter and introns of the motilin (*MLN*) gene, as indicated in Table 5.

**Table 5: Analysis of markers TIHO LDA and amplificates generated by PCR using the primers indicated in Table 3.**

| Marker | SNP / microsatellite | Location of SNP in *MLN* |
|---|---|---|
| TIHO_LDA1 | FN298674:g.62G>A | promoter |
| TIHO_LDA2 | NW001494146:g.560347A>G | 5'UTR |
| TIHO_LDA3 | NW001494146:g.558022insC | intron 1 |
| TIHO_LDA4 | NW001494146:g.557866G>C | intron 1 |
| TIHO_LDA5 | NW001494146:g.554975insA | intron 2 |
| TIHO_LDA6 | NW001494146:g.553228G>A | intron 4 |
| TIHO_LDA7 | NW001494146:g.553189C>T | intron 4 |

Localisation and SNP nomenclature for TIHO_LDA2 to TIHO_LDA7 are given in accordance with Btau 4.0, for TIHO_LDA1 in accordance with NCBI No. 280860.

From the detailed statistics obtained from the analysis of the genotypes of the sampled animals, the data for polymorphism information content (PIC) and heterozygosity (HET), as well as minor allele frequencies (MAF) for LDA-cases and control animals, and odds ratios (OR) per allele and in mutated homozygous vs. wildtype homozygous animals and their corresponding error probabilities (P) for each marker could be determined for 1166 German Holstein cows and are compiled in Table 6 below. For evaluation of the genetic predisposition of an individual animal, these statistic values can be used in a process for breeding cattle, the process including the step of evaluating, e.g. calculating, the genetic disposition for LDA of an individual bovine based on the presence or absence of mutations in the markers of the invention.

**Table 6: PIC, HET, and χ² for the distribution of genotype, alleles and trend in alleles, and corresponding error probabilities for each marker**

| Marker | MAF (LDA) | MAF (controls) | PIC | HET | per allele OR (95% CI) | Homozyg. OR (95% GI) | p-value per allele of OR (-log₁₀) | p-value per allele of homozyg. OR (-log₁₀) |
|---|---|---|---|---|---|---|---|---|
| TIHO_LDA1 | 0.51 | 0.40 | 0.37 | 0.48 | 0.69 (0.58-0.81) | 0.45 (0.32-0.64) | 4.33 | 9.05 |
| TIHO_LDA2 | 0.44 | 0.55 | 0.38 | 0.51 | 0.64 (0.55-0.76) | 0.40 (0.28-0.57) | 6.43 | 13.85 |
| TIHO_LDA3 | 0.56 | 0.42 | 0.38 | 0.47 | 1.75 (1.47-2.09) | 2.98 (2.10-4.23) | 9.65 | 17.73 |
| TIHO_LDA4 | 0.52 | 0.41 | 0.37 | 0.44 | 1.59 (1.33-1.91) | 2.40 (1.69-3.40) | 6.41 | 11.60 |
| TIHO_LDA5 | 0.35 | 0.47 | 0.37 | 0.47 | 1.64 (1.37-1.97) | 2.41 (1.66-3.49) | 6.99 | 10.46 |
| TIHO_LDA6 | 0.28 | 0.40 | 0.34 | 0.36 | 0.58 (0.46-0.73) | 0.37 (0.23-0.58) | 5.40 | 9.09 |
| TIHO_LDA7 | 0.28 | 0.40 | 0.34 | 0.36 | 1.73 (1.37-2.19) | 2.74 (1.72-4.35) | 5.40 | 9.09 |

Further, an interpretation of the data obtained in the genetic analysis resulted in the identification of genotypes for LDA affected cows and non - LDA cows, respectively, and in the identification of the genotype - phenotype relationship. Results are compiled in Table 7.

**Table 7: Genotypes for cows affected by LDA and non-LDA cows**

| SNP | LDA | Control | Genotype-phenotype (LDA) relationship |
|---|---|---|---|
| TIHO_LDA1 | A/A | G/G | recessive |
| TIHO_LDA2 | G/G | A/A | recessive |
| TIHO_LDA3 | insC/insC | WT/insC, WT/WT | recessive |
| TIHO_LDA4 | C/C | G/G | recessive |
| TIHO_LDA5 | insA/insA | WT/insA, WT/WT | recessive |
| TIHO_LDA6 | G/G | A/G, A/A | recessive |
| TIHO_LDA7 | C/C | T/T | recessive |

The analysis of the markers TIHO_LDA1 to TIHO_LDA 7 allows a more precise prediction for an LDA phenotype on the basis of an association test of the haplotype. In Table 9, the frequencies are given for LDA affected cows, and non-LDA (Control) cows, and the statistical analysis for the haplotypes as indicated.

Without TIHO_LDA 3, analysis of the results from genetic testing of markers TIHO_LDA 1 through TIHO_LDA 7 are given in Table 8, for markers TIHO_LDA 2 and TIHO_LDA 3 in Table 9, and for markers TIHO_LDA 1, TIHO_LDA 2 and TIHO_LDA 3 in Table 10.

**Table 8: Haplotype based association tests using markers TIHO LDA1 to TIHO LDA 7**

| Haplotype for markers | Frequency in LDA affected cows | Frequency in control cows | χ² | Error probability P |
|---|---|---|---|---|
| 4-3-5-2-5-1-4 | 0.00 | 0.03 | 45.1381 | <.0001 |
| 4-3-6-3-6-3-2 | 0.45 | 0.29 | 53.3156 | <.0001 |
| 2-3-6-2-6-3-2 | 0.06 | 0.03 | 11.4053 | <.0001 |
| 2-1-5-2-5-3-2 | 0.05 | 0.08 | 6.8685 | .0088 |
| 2-1-5-2-6-1-4 | 0.01 | 0.03 | 22.6019 | <.0001 |
| 2-1-6-3-6-3-2 | 0.01 | 0.03 | 29.4765 | <.0001 |

Ordering of the alleles in the haplotype was done according to ascending numbers of SNP genotypes.

Legend for abbreviations used for the haplotypes:
1: A
2: C
3: G
4: T
5: wt (no insertion in comparison to the mutant allele)
6: insC for TIHO_LDA3 or insA for TIHO_LDA5

**Table 9: Haplotype based association tests using markers TIHO LDA 2, and TIHO LDA 3**

| Haplotype | Frequency in LDA affected cows | Frequency in control cows | χ² | Error probability P |
|---|---|---|---|---|
| 3-5 | 0.01 | 0.07 | 65.3870 | <.0001 |
| 3-6 | 0.55 | 0.38 | 63.1251 | <.0001 |
| 1-5 | 0.44 | 0.51 | 10.8273 | .0010 |
| 1-6 | 0.00 | 0.04 | 42.5333 | <.0001 |

Ordering of the alleles in the haplotype was done according to ascending numbers of SNP genotypes.

Legend for abbreviations used for the haplotypes:
1: A
3:G
5: wt (no insertion in comparison to the mutant allele)
6: insC for TIHO_LDA3

**Table 10: Haplotype based association tests using markers TIHO LDA 1. TIHO_LDA 2, and TIHO LDA 3**

| Haplotype | Frequency in LDA affected cows | Frequency in control cows | χ² | Error probability P |
|---|---|---|---|---|
| 4-3-5 | 0.01 | 0.06 | 52.6356 | <.0001 |
| 4-3-6 | 0.47 | 0.33 | 41.0845 | <.0001 |
| 2-3-5 | 0.00 | 0.01 | 13.1461 | <.0001 |
| 2-3-6 | 0.08 | 0.05 | 11.2928 | .0008 |
| 2-1-5 | 0.43 | 0.50 | 11.4478 | .0007 |
| 2-1-6 | 0.00 | 0.04 | 43.4978 | <.0001 |

Ordering of the alleles in the haplotype was done according to ascending numbers of SNP genotypes.

Legend for abbreviations used for the haplotypes:
1: A
2: C
3: G
4: T
5: wt (no insertion in comparison to the mutant allele)
6: insC for TIHO_LDA3

It can be taken from the above analytical results that the mutant alleles of the markers are coupled to an LDA-phenotype, whereas wild-type alleles are coupled to a non-LDA phenotype.

### Example: Analysis of markers TIHO_LDA by RFLP restriction or sequencing of PCR amplificates containing markers

In this example, analytical results are shown on PCR amplificates which were generated on total genomic DNA obtained from different cows with or without LDA, using the primers as indicated in Table 3 for amplifying DNA fragments containing the respective markers as indicated in the figures.

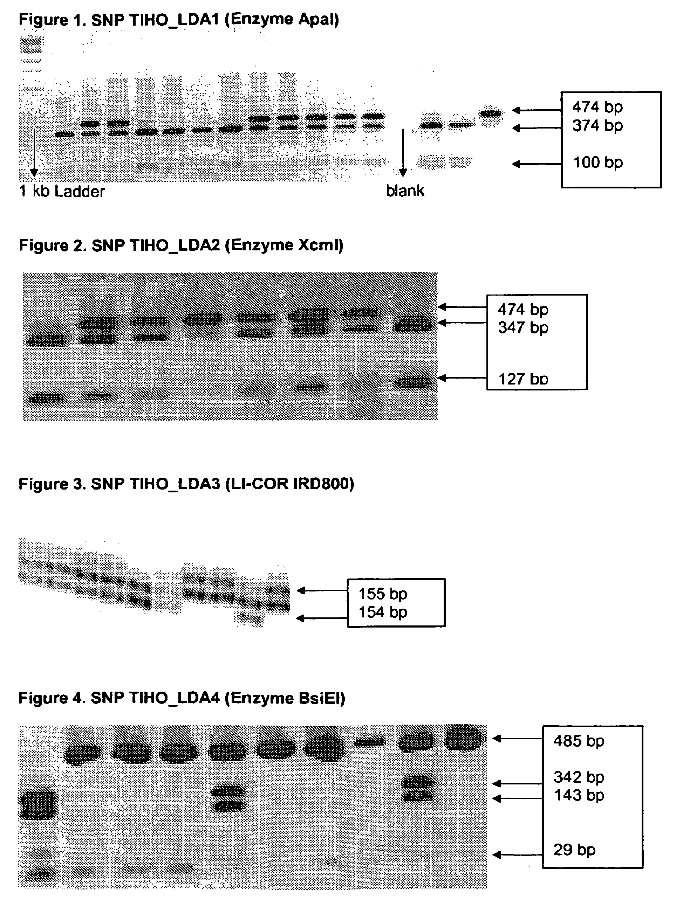

Figure 1 shows the gel-electrophoretic separation of fragments generated by the digestion using ApaI of the amplificate generated by primers LDA 1-f and LDA 1-r. The sizes indicated in base pairs (bp) show the characteristic fragments as given in Table 2, especially the fragment of 474 bp for the LDA - coupled mutant sequence.
Figure 2 shows the gel-electrophoretic separation of fragments, including the fragment of 474 bp characteristic for the LDA - associated mutant sequence of mut-TIHO_LDA 2.
Figure 3 shows the gel-electrophoretic separation ofa sequencing reaction of the PCR amplificate generated using primers LDA 3-f and LDA 3-r , the product comprising marker TIHO_LDA 3, showing the fragment of 154 bp for the non-LDA allele of the marker TIHO_LDA 3, and the 155 bp of mut-TIHO_LDA 3, which is coupled with LDA. The band occurring between the indicated 155 bp and 154 bp is a non-specific shadow.
Figure 4 shows the gel-electrophoretic separation of restriction fragments of the amplificate generated using primers LDA 4-f and LDA 4-r, the product comprising marker TIHO_LDA 4, showing the fragment of 458 bp for the non-LDA allele of the marker TIHO LDA 4, which is missing in mut-TIHO_LDA4.

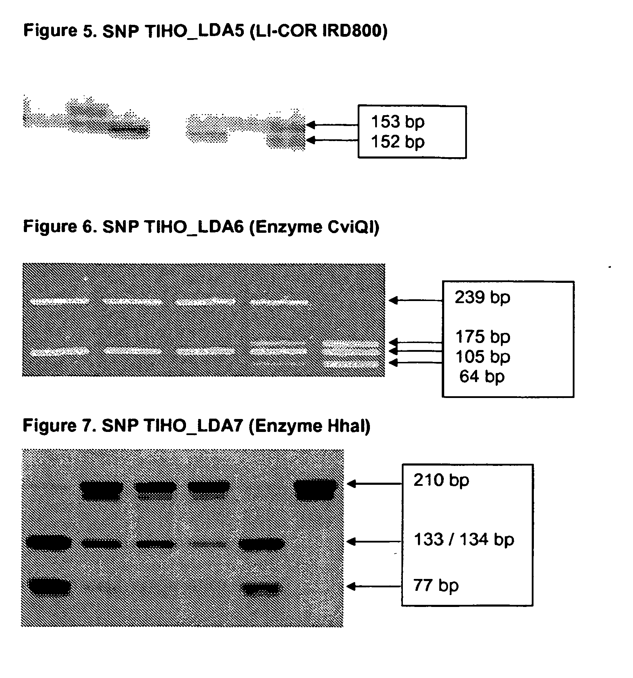

Figure 5 shows the gel-electrophoretic separation of a sequencing reaction of the PCR amplificate generated using primers LDA 5-f and LDA 5-r , the product comprising marker TIHO_LDA 5, showing the fragment of 152 bp for the non-LDA allele of the marker TIHO_LDA 5, and the 153 bp of mut-TIHO_LDA5.
Figure 6 shows the gel-electrophoretic separation of restriction fragments of the amplificate generated using primers LDA 6-f and LDA 6-r , the product comprising marker TIHO_LDA 6, showing the fragment of 175 bp for the non-LDA allele of the marker TIHO_LDA 6, and the fragment of 239 bp in mut-TIHO_LDA6.
Figure 7 shows the gel-electrophoretic separation of restriction fragments of the amplificate generated using primers LDA 7-f and LDA 7-r , the product comprising marker TIHO_LDA 7, showing the fragment of 77 bp for the non-LDA allele of the marker TIHO_LDA 7, and the fragment of 210 bp in mut-TIHO_LDA7.

As a specific example for one LDA-affected cow and a non-affected cow, the following set of alleles were determined in total genomic DNA using the marker-specific primers as given herein in PCR:

| SNP | LDA-affected | Control |
|---|---|---|
| TIHO_LDA1 | A/A | G/G |
| TIHO_LDA2 | G/G | A/A |
| TIHO_LDA3 | insC/insC | WT/WT |
| TIHO_LDA4 | C/C | G/G |
| TIHO_LDA5 | insA/insA | WT/WT |
| TIHO_LDA6 | G/G | A/A |
| TIHO_LDA7 | C/C | T/T |

## Claims

1. Process for analysis of the genetic disposition of cattle for LDA, **characterized by** the detection of at least one aberration in the bovine motilin gene in at least one of the markers contained in the group consisting of Seq ID No. 1 (TIHO_LDA 1), Seq ID No. 2 (TIHO_LDA 2), Seq ID No. 3 (TIHO_LDA 3), Seq ID No. 4 (TIHO_LDA 4), Seq ID No. 5 (TIHO_LDA 5), Seq ID No. 6 (TIHO_LDA 6), Seq ID No. 7 (TIHO_LDA 7), and nucleic acid sequences having a sequence that is reverse complementary to these sequences.

2. Process according to claim 1, **characterized in that**
Seq ID No. 1 (TIHO_LDA 1) is contained in the PCR amplificate obtained by primers
Seq ID No. 15 (LDA 1-f) and Seq ID No. 16 (LDA 1-r),
Seq ID No. 2 (TIHO_LDA 2) is contained in the PCR amplificate obtained by primers
Seq ID No. 17 (LDA 2-f) and Seq ID No. 18 (LDA 2-r),
Seq ID No.3 (TIHO_LDA 3) is contained in the PCR amplificate obtained by primers
Seq ID No. 19 (LDA 3-f) and Seq ID No. 20 (LDA 3-r),
Seq ID No. 4 (TIHO_LDA 4) is contained in the PCR amplificate obtained by primers
Seq ID No. 21 (LDA 4-f) and Seq ID No. 22 (LDA 4-r),
Seq ID No. 5 (TIHO_LDA 5) is contained in the PCR amplificate obtained by primers
Seq ID No. 23 (LDA 5-f) and Seq ID No. 24 (LDA 5-r),
Seq ID No. 6 (TIHO_LDA 6) is contained in the PCR amplificate obtained by primers
Seq ID No. 25 (LDA 6-f) and Seq ID No. 26 (LDA 6-r), and
Seq ID No. 7 (TIHO_LDA 7) is contained in the PCR amplificate obtained by primers
Seq ID No. 27 (LDA 7-f) and Seq ID No. 28 (LDA 7-r).

3. Process according to one of the preceding claims, **characterized in that**
the aberration of Seq ID No. 1 (TIHO_LDA 1) is Seq ID No. 8 (mut-TIHO_LDA 1),
the aberration of Seq ID No. 2 (TIHO_LDA 2) is Seq ID No. 9 (mut-TIHO_LDA 2),
the aberration of Seq ID No. 3 (TIHO_LDA 3) is Seq ID No. 10 (mut-TIHO_LDA 3) the aberration of Seq ID No. 4 (TIHO_LDA 4) is Seq ID No. 11 (mut-TIHO_LDA 4), the aberration of Seq ID No. 5 (TIHO_LDA 5) is Seq ID No. 12 (mut-TIHO_LDA 5),
the aberration of Seq ID No. 6 (TIHO_LDA 5) is Seq ID No. 13 (mut-TIHO_LDA 6), and
the aberration of Seq ID No. 7 (TTHO_LDA 7) is Seq ID No. 14 (mut-TIHO_LDA 7).

4. Process according to one of the preceding claims, **characterized by** the detection of the marker in total genomic DNA of an individual cattle.

5. Process according to one of the preceding claims, **characterized by** selecting an individual cattle for breeding.

6. Process according to one of the preceding claims, **characterized in that** the aberration in the bovine motilin gene is in the non-translated sequences of the bovine motilin gene.

7. Process according to one of the preceding claims, **characterized in that** at least the aberration of marker Seq ID No. 3 (TIHO_LDA 3) or of its reverse complementary sequence is analysed.

8. Process according to one of the preceding claims, **characterized in that** at least the aberration of marker Seq ID No. 2 (TTHO_LDA 2) and of marker Seq ID No. 3 (TIHO_LDA 3) or of a reverse complementary sequence thereof is analysed.

9. Process according to one of the preceding claims, **characterized in that** the aberration of markers Seq ID No. 1 (TIHO_LDA 1), Seq ID No. 2 (TIHO_LDA 2), Seq ID No. 3 (TIHO_LDA 3), Seq ID No. 4 (TIHO_LDA 4), Seq ID No. 5 (TIHO_LDA 5), Seq ID No. 6 (TIHO_LDA 6). Seq ID No. 7 (TIHO_LDA 7), or of a reverse complementary sequence thereof is analysed.

10. Genetic marker for use in the analysis of the genetic disposition for LDA of cattle by a process according to one of the preceding claims, **characterized in that** the genetic marker comprises a sequence contained in the group consisting of Seq ID No. 1 (TIHO_LDA 1), Seq ID No. 8 (mut-TIHO_LDA 1), Seq ID No. 2 (TIHO_LDA 2), Seq ID No. 9 (mut-TIHO_LDA 2), Seq ID No. 3 (TIHO_LDA 3), Seq ID No. 10 (mut-TIHO_LDA 3), Seq ID No. 4 (TIHO_LDA 4), Seq ID No. 11 (mut-TIHO_LDA 4). Seq ID No. 5 (TIHO_LDA 5), Seq ID No. 12 (mut-TIHO_LDA 5). Seq ID No. 6 (TIHO_LDA 5), Seq ID No. 13 (mut-TIHO_LDA 6), Seq ID No. 7 (TIHO_LDA 7), Seq ID No. 14 (mut-TIHO_LDA 7), and nucleic acid sequences having a sequence that is reverse complementary to these sequences.

11. Genetic marker according to claim 10, **characterized in that** the genetic marker is comprised in a PCR amplificate selected from the group consisting of nucleic acid sections of the bovine motilin gene consisting of PCR amplificates obtained with pairs of primers having Seq ID No. 15 (LDA 1-f) and Seq ID No. 16 (LDA 1-r), or primers Seq ID No. 17 (LDA 2-f) and Seq ID No. 18 (LDA 2-r ), or Seq ID No. 19 (LDA 3-f) and Seq ID No. 20 (LDA 3-r), or Seq ID No. 21 (LDA 4-f) and Seq ID No. 22 (LDA 4-r), or Seq ID No. 23 (LDA 5-f) and Seq m No. 24 (LDA 5-r ), or Seq ID No. 25 (LDA 6-f) and Seq ID No. 26 (LDA 6-r ), or Seq ID No. 27 (LDA 7-f) and Seq ID No. 28 (LDA 7-r), and nucleic acid sequence pairs having sequences that are reverse complementary to these pairs.

12. Oligonucleotide for use as a primer for the synthesis of an amplificate from total genomic bovine DNA, selected from the group consisting of Seq 4) No. 1 (LDA 1-f), Seq ID No. 16 (LDA 1-r), Seq ID No. 17 (LDA 2-f). Seq ID No. 18 (LDA 2-r ), Seq ID No. 19 (LDA 3-f), Seq ID No. 20 (LDA 3-r ), Seq ID No. 21 (LDA 4-f), Seq ID No. 22 (LDA 4-r ), Seq ID No. 23 (LDA 5-f), Seq ID No. 24 (LDA 5-r), Seq ID No. 25 (LDA 6-f), Seq ID No. 26 (LDA 6-r ), Seq ID No. 27 (LDA 7-f), Seq ID No. 28 (LDA 7-r ), and nucleic acid sequences having a sequence that is reverse complementary to these sequences.

## Patentansprüche

1. Verfahren zur Analyse der genetischen Disposition von Rindern auf LDA,
**gekennzeichnet durch** die Detektion zumindest einer Aberration im Motilingen des Rindes in zumindest einem der Marker, die in der Gruppe enthalten sind, die aus Seq ID No. 1 (TIHO_LDA 1), Seq ID No. 2 (TIHO_LDA 2), Seq ID No. 3 (TIHO_LDA 3), Seq ID No. 4 (TIHO_LDA 4), Seq ID No. 5 (TIHO_LDA 5), Seq ID No. 6 (TIHO_LDA 6), Seq ID No. 7 (TIHO_LDA 7), und Nukleinsäuresequenzen, die eine zu diesen Sequenzen revers komplementäre Sequenz aufweist, besteht.

2. Verfahren nach Anspruch 1, **dadurch gekennzeichnet, dass**
Seq ID No. 1 (TIHO_LDA 1) in dem PCR-Amplifikat enthalten ist, das mit den Primern mit den Seq ID No. 15 (LDA 1-f) und Seq ID No. 16 (LDA 1-r) erhalten wird,
Seq ID No. 2 (TIHO_LDA 2) in dem PCR-Amplifikat enthalten ist, das mit den Primem mit den Seq ID No. 17 (LDA 2-f) und Seq ID No. 18 (LDA 2-r ) erhalten wird,
Seq ID No. 3 (TIHO_LDA 3) in dem PCR-Amplifikat enthalten ist, das mit den Primem mit den Seq ID No. 19 (LDA 3-f) und Seq ID No. 20 (LDA 3-r ) erhalten wird,
Seq ID No. 4 (TIHO_LDA 4) in dem PCR-Amplifikat enthalten ist, das mit den Primem mit den Seq ID No. 21 (LDA 4-f) und Seq ID No. 22 (LDA 4-r ) erhalten wird,
Seq ID No. 5 (TIHO_LDA 5) in dem PCR-Amplifikat enthalten ist, das mit den Primem mit den Seq ID No. 23 (LDA 5-f) und Seq ID No. 24 (LDA 5-r ) erhalten wird,
Seq ID No. 6 (TIHO_LDA 6) in dem PCR-Amplifikat enthalten ist, das mit den Primem mit den Seq ID No. 25 (LDA 6-f) und Seq ID No. 26 (LDA 6-r ) erhalten wird, und
Seq ID No. 7 (TIHO_LDA 7) in dem PCR-Amplifikat enthalten ist, das mit den Primem mit den Seq ID No. 27 (LDA 7-f) und Seq ID No. 28 (LDA 7-r ) erhalten wird.

3. Verfahren nach einem der voranstehenden Ansprüche, **dadurch gekennzeichnet, dass**
die Aberration von Seq ID No. 1 (TIHO_LDA 1) die Seq ID No. 8 (mut-TIHO_LDA 1) aufweist,
die Aberration von ID No. 2 (TIHO_LDA 2) von Seq ID No. 9 (mut-TIHO_LDA 2) aufweist,
die Aberration von ID No. 3 (TIHO_LDA 3) die Seq ID No. 10 (mut-TIHO_LDA 3) aufweist,
die Aberration von Seq ID No. 4 (TIHO_LDA 4) die Seq ID No. 11 (mut-TIHO_LDA 4) aufweist,
die Aberration von Seq ID No. 5 (TIHO_LDA 5) die Seq ID No. 12 (mut-TIHO_LDA 5) aufweist,
die Aberration von Seq ID No. 6 (TIHO_LDA 5) die Seq ID No. 13 (mut-TIHO_LDA 6) aufweist, und
die Aberration von Seq ID No. 7 (TIHO_LDA 7) die Seq ID No. 14 (mut-TIHO_LDA 7) aufweist.

4. Verfahren nach einem der voranstehenden Ansprüche, **gekennzeichnet durch** die Detektion des Markers in genomischer Gesamt-DNA eines einzelnen Rindes.

5. Verfahren nach einem der voranstehenden Ansprüche, **gekennzeichnet durch** das Auswählen eines einzelnen Rindes zur Zucht.

6. Verfahren nach einem der voranstehenden Ansprüche, **dadurch gekennzeichnet, dass** die Aberration im Motilingen des Rindes in den nicht-translatierten Sequenzen des Motilingens des Rindes ist.

7. Verfahren nach einem der voranstehenden Ansprüche, **dadurch gekennzeichnet, dass** die Aberration des Markers Seq ID No. 3 (TIHO_LDA 3) oder dessen revers komplementärer Sequenz analysiert wird.

8. Verfahren nach einem der voranstehenden Ansprüche, **dadurch gekennzeichnet, dass** zumindest die Aberration des Markers der Seq ID No. 2 (TIHO_LDA 2) und des Markers der Seq ID No. 3 (TIHO_LDA 3) oder einer dazu revers komplementären Sequenz analysiert wird.

9. Verfahren nach einem der voranstehenden Ansprüche, **dadurch gekennzeichnet, dass** die Aberration der Marker der Seq ID No. 1 (TIHO_LDA 1), Seq ID No. 2 (TIHO_LDA 2), Seq ID No. 3 (TIHO_LDA 3), Seq ID No. 4 (TIHO_LDA 4), Seq ID No. 5 (TIHO_LDA 5), Seq ID No. 6 (TIHO_LDA 6), Seq ID No. 7 (TIHO_LDA 7), oder einer dazu revers komplementären Sequenz analysiert wird.

10. Genetischer Marker zur Verwendung in der Analyse der genetischen Disposition von Rindern zur LDA durch ein Verfahren nach einem der voranstehenden Ansprüche, **dadurch gekennzeichnet, dass** der genetische Marker eine Sequenz umfasst, die in der Gruppe enthalten ist, die aus Seq ID No. 1 (TIHO_LDA 1), Seq ID No. 8 (mut-TIHO_LDA 1), Seq ID No. 2 (TIHO_LDA 2), Seq ID No. 9 (mut-TIHO_LDA 2), Seq ID No. 3 (TIHO_LDA 3), Seq ID No. 10 (mut-TIHO_LDA 3), Seq ID No. 4 (TIHO_LDA 4), Seq ID No. 11 (mut-TIHO_LDA 4), Seq ID No. 5 (TIHO_LDA 5), Seq ID No. 12 (mut-TIHO_LDA 5), Seq ID No. 6 (TIHO_LDA 5), Seq ID No. 13 (mut-TIHO_LDA 6), Seq ID No. 7 (TIHO_LDA 7), Seq ID No. 14 (mut-TIHO_LOA 7) und Nukleinsäuren besteht, die eine Sequenz haben, die eine zu diesen Sequenzen revers komplementäre Sequenz haben.

11. Genetischer Marker nach Anspruch 10, **dadurch gekennzeichnet, dass** der genetische Marker in einem PCR-Amplifikat enthalten ist, das aus der Gruppe ausgewählt ist, die aus Nukleinsäureabschnitten des Motilingens des Rindes besteht, die aus PCR-Amplifikaten besteht, die mit den Primerpaaren mit Seq ID No. 15 (LDA 1-f) und Seq ID No. 16 (LDA 1-r), oder Primern Seq ID No. 17 (LDA 2-f) und Seq ID No. 18 (LDA 2-r), oder Seq ID No. 19 (LDA 3-f) und Seq ID No. 20 (LDA 3-r), oder Seq ID No. 21 (LDA 4-f) und Seq ID No. 22 (LDA 4-r), oder Seq ID No. 23 (LDA 5-f) und Seq ID No. 24 (LDA 5-r), oder Seq ID No. 25 (LDA 6-f) und Seq ID No. 26 (LDA 6-r ), oder Seq ID No. 27 (LDA 7-f) und Seq ID No. 28 (LDA 7-r) und Paaren von Nukleinsäuresequenzen, die zu diesen Paaren revers komplementäre Sequenzen aufweisen, erhalten sind.

12. Oligonukleotid zur Verwendung als ein Primer für die Synthese eines Amplifikats aus genomischer Gesamt-DNA des Rindes, ausgewählt aus der Gruppe, die aus Seq ID No. 15 (LDA 1-f), Seq ID No. 16 (LDA 1-r), Seq ID No. 17 (LDA 2-f), Seq ID No. 18 (LDA 2-r), Seq ID No. 19 (LDA 3-f), Seq ID No. 20 (LDA 3-r), Seq ID No. 21 (LDA 4-f), Seq ID No. 22 (LDA 4-r), Seq ID No. 23 (LDA 5-f), Seq ID No. 24 (LDA 5-r), Seq ID No. 25 (LDA 6-f), Seq ID No. 26 (LDA 6-r), Seq ID No. 27 (LDA 7-f), Seq ID No. 28 (LDA 7-r) und Nukleinsäuresequenzen, die eine zu diesen Sequenzen revers komplementäre Sequenz haben, besteht

## Revendications

1. Procédé d'analyse de la disposition génétique du bétail au DCG, **caractérisé par** la détection d'au moins une aberration dans le gène bovin de la motiline dans au moins l'un des marqueurs contenus dans le groupe constitué de Seq ID No. 1 (TIHO_LDA 1), Seq ID No. 2 (TIHO-LDA 2), Seq ID No. 3 (TIHO_LDA 3), Seq ID No. 4 (TIHO_LDA 4), Seq ID No. 5 (TIHO_LDA 5), Seq ID No. 6 (TIHO_LDA 6), Seq ID No. 7 (TIHO_LDA 7), et de séquences d'acides nucléiques ayant une séquence inversement complémentaire à ces séquences.

2. Procédé selon la revendication 1, **caractérisé par le fait que**
Seq ID No. 1 (TIHO_LDA 1) est contenu dans l'amplification par PCR obtenue par les amorces Seq ID No. 15 (LDA 1-f) et Seq ID No. 16 (LDA 1-r),
Seq ID No. 2 (TIHO_LDA 2) est contenu dans l'amplification par PCR obtenue par les amorces Seq ID No. 17 (LDA 2-f) et Seq ID No. 18 (LDA 2-r),
Seq ID No. 3 (TIHO_LDA 3) est contenu dans l'amplification par PCR obtenue par les amorces Seq ID No. 19 (LDA 3-f) et Seq ID No. 20 (LDA 3-r),
Seq ID No. 4 (TIHO_LDA 4) est contenu dans l'amplification par PCR obtenue par les amorces Seq ID No. 21 (LDA 4-f) et Seq ID No. 22 (LDA 4-r),
Seq ID No. 5 (TIHO_LDA 5) est contenu dans l'amplification par PCR obtenue par les amorces Seq ID No. 23 (LDA 5-f) et Seq ID No. 24 (LDA 5-r),
Seq ID No. 6 (TIHO-LDA 6) est contenu dans l'amplification par PCR obtenue par les amorces Seq ID No. 25 (LDA 6-f) et Seq ID No. 26 (LDA 6-r), et
Seq ID No. 7 (TIHO_LDA 7) est contenu dans l'amplification par PCR obtenue par les amorces Seq ID No. 27 (LDA 7-f) et Seq ID No. 28 (LDA 7-r).

3. Procédé selon l'une des revendications précédentes, **caractérisé par le fait que**
l'aberration de Seq ID No. 1 (TIHO_LDA 1) est Seq ID No. 8 (mut-TIHO_LDA 1),
l'aberration de Seq ID No. 2 (TIHO_LDA 2) est Seq ID No. 9 (mut-TIHO_LDA 2),
l'aberration de Seq ID No. 3 (TIHO_LDA 3) est Seq ID No. 10 (mut-TIHO_LDA 3),
l'aberration de Seq ID No. 4 (TIHO_LDA 4) est Seq ID No. 11 (mut-TIHO_LDA 4),
l'aberration de Seq ID No. 5 (TIHO_LDA 5) est Seq ID No. 12 (mut-TIHO_LDA 5),
l'aberration de Seq ID No. 6 (TIHO_LDA 5) est Seq ID No. 13 (mut-TIHO_LDA 6),
et l'aberration de Seq ID No. 7 (TIHO_LDA 7) est Seq ID No. 14 (mut-TIHO_LOA 7).

4. Procédé selon l'une des revendications précédentes, **caractérisé par** la détection du marqueur dans un ADN génomique total d'un bétail individuel.

5. Procédé selon l'une des revendications précédentes, **caractérisé par** la sélection d'un bétail individuel pour la reproduction.

6. Procédé selon l'une des revendications précédentes, **caractérisé par le fait que** l'aberration dans le gène bovin de la motiline est dans les séquences non transcrites du gène bovin de la motiline.

7. Procédé selon l'une des revendications précédentes, **caractérisé par le fait qu'**au moins l'aberration du marqueur Seq ID No. 3 (TIHO_LDA 3) ou de sa séquence inversement complémentaire est analysée.

8. Procédé selon l'une des revendications précédentes, **caractérisé par le fait qu'**au moins l'aberration du marqueur Seq ID No. 2 (TIHO_LDA 2) et du marqueur Seq ID No. 3 (TIHO-LDA 3) ou d'une séquence inversement complémentaire de ceux-ci est analysée.

9. Procédé selon l'une des revendications précédentes, **caractérisé par le fait que** l'aberration des marqueurs Seq ID No. 1 (TIHO_LDA 1), Seq ID No. 2 (TIHO_LOA 2), Seq ID No. 3 (TIHO_LDA 3), Seq ID No. 4 (TIHO_LDA 4), Seq ID No. 5 (TIHO_LDA 5), Seq ID No. 6 (TIHO_LDA 6), Seq ID No. 7 (TIHO_LDA 7), ou d'une séquence inversement complémentaire de ceux-ci est analysée.

10. Marqueur génétique destiné à être utilisé dans l'analyse de la disposition génétique au DCG du bétail par un procédé selon l'une des revendications précédentes, **caractérisé par le fait que** le marqueur génétique comprend une séquence contenue dans le groupe constitué de Seq ID No. 1 (TIHO_LDA 1), Seq ID No. 8 (mut-TIHO_LDA 1), Seq ID No. 2 (TIHO_LDA 2), Seq ID No. 9 (mut-TIHO_LDA 2), Seq ID No. 3 (TIHO_LDA 3), Seq ID No. 10 (mut-TIHO_LDA 3), Seq ID No. 4 (TIHO_LDA 4), Seq ID No. 11 (mut-TIHO_LDA 4), Seq ID No. 5 (TIHO_LDA 5), Seq ID No. 12 (mut-TIHO_LDA 5), Seq ID No. 6 (TIHO_LDA 5), Seq ID No. 13 (mut-TIHO_LDA 6), Seq ID No. 7 (TIHO_LDA 7), Seq ID No. 14 (mut-TIHO_LDA 7), et des séquences d'acide nucléique ayant une séquence qui est inversement complémentaire à ces séquences.

11. Marqueur génétique selon la revendication 10, **caractérisé par le fait que** le marqueur génétique est compris dans une amplification par PCR choisie parmi le groupe constitué de sections d'acide nucléique du gène bovin de la motiline constitué d'amplifications par PCR obtenues avec des paires d'amorce ayant des Seq ID No. 15 (LDA 1-f) et Seq ID No. 16 (LDA 1-r), ou des amorces Seq ID No. 17 (LDA 2-f) et Seq ID No. 18 (LDA 2-r), ou Seq ID No. 19 (LDA 3-f) et Seq ID No. 20 (LDA 3-r), ou Seq ID No. 21 (LDA 4-f) et Seq ID No. 22 (LDA 4-r), ou Seq ID No. 23 (LDA 5-f) et Seq ID No. 24 (LDA 5-r), ou Seq ID No. 25 (LDA 6-f) et Seq ID No. 26 (LDA 6-r), ou Seq ID No. 27 (LDA 7-f) et Seq ID No. 28 (LDA 7-r), et des paires de séquences d'acide nucléique ayant des séquences qui sont inversement complémentaires à ces paires.

12. Oligonucléotide destiné à être utilisé comme une amorce pour la synthèse d'une amplification depuis l'ADN génomique bovin total, choisi parmi le groupe constitué de Seq ID No. 15 (LDA 1-f), Seq ID No. 16 (LDA 1-r), Seq ID No. 17 (LDA 2-f), Seq ID No. 18 (LDA 2-r), Seq ID No. 19 (LDA 3-f), Seq ID No. 20 (LDA 3-r), Seq ID No. 21 (LDA 4-f), Seq ID No. 22 (LDA 4-r), Seq ID No. 23 (LDA 5-f), Seq ID No. 24 (LDA 5-r), Seq ID No. 25 (LDA 6-f), Seq ID No. 26 (LDA 6-r), Seq ID No. 27 (LDA 7-f), Seq ID No. 28 (LDA 7-r), et des séquences d'acide nucléique ayant une séquence qui est inversement complémentaire à ces séquences.
